# EUROPEAN PATENT APPLICATION

(11) **EP 1 867 321 A2**
(43) Date of publication of application: **19.12.2007**
(21) Application number: 07109714.1
(22) Date of filing: 06.06.2007
(51) Int. Cl.: A61K 9/00, A61K 47/48, A61K 31/568, A61K 31/565, A61K 31/573

(54) **A pharmaceutical product adapted for oral transmucosal administration comprising a pharmaceutical agent**

(30) Priority: 07.06.2006 EP 06115066
(71) Applicant: Familplan Consulting Ltd., 01531 Vantaa (FI)
(72) Inventor: Luukkainen, Tapani, 02160, Espoo (FI)
(74) Representative: Suominen, Kaisa Liisa

(57) **Abstract**

The invention relates to a pharmaceutical product adapted for oral transmucosal administration in the form of filtration paper for sublingual use comprising a water-soluble complex of a pharmaceutical agent and a cyclodextrin derivative adsorbed on said filtration paper, wherein the pharmaceutical agent is estradiol or testosterone in an amount of 0.01 - 7 mg, or cortisone or a synthetic potent analogues of cortisone in an amount that is clinically effective.

## Description

The invention relates to a pharmaceutical product adapted for oral transmucosal administration comprising a water-soluble complex of a pharmaceutical agent and a cyclodextrin derivative and to uses relating thereto.

### BACKGROUND OF THE INVENTION

Human sex hormones have been identified and their role in the body functions has been studied extensively. It is known that supplementation with sex hormones, such as androgens and estrogens, has a potential to ameliorate some problems stemming from body wasting diseases and from aging.

However, numerous negative side effects prevent a wide use of such supplementations. When supplemented through gastrointestinal tract the hormones are rapidly metabolised by the liver, making a standard oral pill administration inefficient. This problem has been solved by the use of metabolism resisting unnatural analogs or parenteral depo formulations. However, some side effects still persist.

The level of sex hormones varies in healthy subjects. For example, in male mammals, testosterone is present in circulation at relatively low level at all times and small variations from the basal level occur in term of minutes. Additionally, large increases, called pulses, occur multiple times per hour or per day. The serum levels of sex hormones are regulated in a complex manner. Diseases, postnatal changes or aging may affect overall hormone levels.

The general belief is that for therapeutic purposes the level of the sex hormone in the body ought to be augmented to significantly high and prolonged levels. For example, widely used supplementation of testosterone is based on intramuscular injections of oily solution of testosterone esters, e.g. enanthate, from which testosterone is slowly released. This treatment strongly increases level of hormone for approximately a week, whereby it gradually decreases until the next injection is administered. Supplementations of steroid hormone by transdermal routes are administered in shorter intervals, but also these pharmaceutical forms supply the hormone into circulation in considerable amounts raising the hormone level clearly above the natural basal level.

Sex hormones form water-soluble complexes with cyclodextrin derivatives. Cyclodextrins are oligosaccharides in which six, seven or eight glucose residues are joined into a cycle by glycosidic bonds. Beta-cyclodextrin has seven glucose residues. Derivatisation of some of the hydroxyls of a cyclodextrin, for example, by methyl or hydroxypropyl groups, leads to mixtures, called cyclodextrin derivatives, which have, compared to the starting cyclodextrin, higher water solubility.

US 4,596,795 discloses administration of sex hormones in the form of hydrophilic cyclodextrin derivatives. It is stated that a daily dosage of 0.1 - 25 mg sex hormone can be administered by injecting by the buccal route, and sublingual administration can comprise 10 and 14 mg of testosterone cyclodextrin derivative complex. These high dosages elevate the hormone levels in serum for several hours over the basal level.

US 6,884,790 discloses a solid dosage form that does not disintegrate in water and contains a drug in form of inclusion complex with a cyclodextrin. It is stated that steroid hormones are typically administered in up to 10 mg doses.

It is further to be noted that before advent of tablets, a presently forgotten dosage form, medicated papers, was in wide use. The principle is sound: if drug dissolves easily and fast in water, its solution may be dried into blotting paper and, when this paper comes into contact with saliva, drug dissolves and is eluted from paper. Medicated papers of the past were, in reality, never effective. Very few drugs alone are soluble enough in water and effective enough for this old approach to work. Additionally, drying of drug solutions often forms crystals that can be shaken out of medicated paper.

Another problem encountered in the field of pharmaceutics is the allergic reactions to for example a bite of a wasps or a snake, or to some other allergen, such as a particular food. Some persons can have severe allergic reactions that may be lethal if they cannot have an injection of cortisone quickly. This can be a major problem in places located far from hospitals. Typically persons that know they can have severe allergic reactions have a syringe filled with cortisone with them at all times. Some people are however not comfortable with injecting themselves and such filled syringe can be difficult to carry, not to mention the increased security issues concerning sharp items.

### OBJECT OF THE INVENTION

The object of the invention is to minimise or even eliminate the problems existing in the prior art.

An object of the present invention is to provide a safe pharmaceutical composition for administering human sex hormones.

Another object of the invention is to minimise the adverse side effect associated with conventional sex hormone administration.

A further object of the invention is to provide a convenient product for the administration of cortisone to persons suffering from allergic reactions.

### SUMMARY OF THE INVENTION

In order to achieve the above-mentioned objects the present invention is characterised in what is defined in the characterising parts of the independent claims presented hereafter.

Typical pharmaceutical product adapted for oral transmucosal administration in the form of filtration paper for sublingual use comprising a water-soluble complex of a pharmaceutical agent and a cyclodextrin derivative adsorbed on said filtration paper, wherein the pharmaceutical agent is
- estradiol or testosterone in an amount of 0.01 - 7 mg, or
- cortisone or a synthetic potent analogue of cortisone in an amount that is clinically effective.

Typically, according to the present invention a complex of a sex hormone and a cyclodextrin derivative is used for manufacture of an oral sublingual supplement, in the form of a filtration paper into which filtration paper said complex has been absorbed, for treatment of hormone depletion, said medicament comprising 0.01 - 7 mg of hormone and said sex hormone being selected from the group consisting of estradiol and testosterone.

According to the present invention a water-soluble complex of cortisone and a cyclodextrin derivative is used for manufacture of an oral transmucosal medicament for treatment of an allergic reaction and/or anaphylactic shock, said medicament comprising cortisone or a synthetic potent analogue of cortisone in an amount that is clinically effective.

### DETAILED DESCRIPTION OF THE INVENTION

Now it has been surprisingly found out that the sex hormones can be administered in very low doses and still be able to achieve significant improvements in subject's well-being. It has been realised that by administering sex hormones in very low levels through oral mucosa it is possible to imitate the natural variation in hormone levels in circulation. The hormone released by the pharmaceutical composition according to the present invention reaches the tissues in a pulse like manner, corresponding to physiological secretion for testis or ovary. Thus the body is exposed for the elevated hormone level only for a short time, which reduces the possibility for negative side effects. The small physiological amount of the hormone that is released also minimise the formation of metabolites, which could cause adverse side effects.

Use of low levels of hormones makes the composition according to the present invention especially suitable for long-term administration, as the risk for negative side effects can be minimised and there is no risk for overdose. The composition is also cheaper to prepare as the amount of active hormone is low in the composition.

In the present context the term sex hormone denotes a natural sex hormone occurring naturally in a healthy human subject. The preferred sex hormones are testosterone and estradiol. These sex hormones can be solubilised by inclusion complexes formed with cyclodextrins and their derivatives. Cyclodextrin derivatives preferably comprise partial methyl, hydroxypropyl or sulfoalkyl ethers, or partial acetyl esters of alpha-, beta- or gamma-cyclodextrins.

This same effect of possibility of administering cortisone through oral mucosa has also been surprisingly discovered. In this description, by cortisone it is meant not only cortisone itself, but also its synthetic potent analogues, even if these are not always expressly mentioned. The administration of cortisone by sublingual filtration paper has the great advantage of achieving a response in the patient as quickly as by injecting the cortisone in a traditional manner. Indeed, the cortisone passes almost immediately to the blood through the oral mucosa and reduces the allergic reaction.

Furthermore, the present invention allows the preparation and use of sublingual products based on filtering paper onto which the complex of a pharmaceutical agent and a cyclodextrin derivative has been adsorbed. This administration means prevents for example the use of testosterone in doping, as the product is preferably shaped such that only one product can be used at a time. This would reduce the interest in misuse, as it would be more complicated and time consuming than traditional injections. A further major advantage of the present invention is the possibility to dose the pharmaceutical agent accurately. Moreover, there is no risk of accidental breaking of the tablet and its subsequent swallowing by the patient.

The present invention also has the further advantage that overdose is less likely than with for example sublingual tablets. There is also a significantly reduced risk of accidental swallowing of the paper, compared to sublingual tablets. Moreover, it is easier and cheaper to produce the filtration paper dosage forms according to the invention that it is to produce tablets. It is also possible to use coloured paper, which allows easy checking of the administration of the pharmaceutical agent. Indeed, when the colour is chosen in an appropriate way, it elutes at substantially the same rate as the pharmaceutical agent and thus de-coloration of the paper indicates that essential of the pharmaceutical agent has eluted at the intended place and rate. According to a preferred embodiment of the invention, the disc of filtration paper is of size that only one fits comfortably into a mouth of the intended patient.

This dosage form also solves a number of production problems and enables continuous processing. For production of this new dosage form, the solutions of complexes of pharmaceutical agents can be directly applied to an absorbent material and dried into it. Use of rolls of absorbent paper enables continuous deposition of the solution and, since wetted paper is semitransparent, the amount and uniformity of the deposition can be continuously screened by photometry. Thus, mandated quality controls can be done on all products rather than just samples (as is required with batches of standard tablets). From a production point of view, the invented process eliminates the need for separate development of formulation methods for solid dosages of different pharmaceutical agents, as required when using previously existing technology. That is, powders of different pharmaceutical agents handle differently, but their solutions in cyclodextrin derivatives pour the same. The same processes and machinery could be used for many different pharmaceutical agents. Cleanup times of machinery between productions of different pharmaceutical agents can be expected much shorteraqueous solutions are easier to wash off than are various powders.

This method could be used for the production of other pharmaceutical products. The old compounds could be converted to fast acting formulations using cyclodextrin derivatives for first aid use in homes and while travelling. They could replace intravenous injections making larger use of pharmaceutical agents presently used in injection because these pharmaceutical agents in sublingual formulations are safer and more convenient in the hands of paramedical personnel. The following areas are practically fruitful for the use of these methods: (1) Cortisone for anaphylactic shock, (2) migraine (injections are inconvenient, current pill formulations induce vomiting), (3) Viagra (sildenafil) type pharmaceutical agents.

Steroid hormones concerned are such powerful medications that they may become dangerous when overused in an abusive manner. Athletes and bodybuilders use testosterone to increase their physical strength and when that illegal abuse is performed in massive ways, natural testicular function in men is decreased and females experience masculinization. Cortisone, when overused by patients, who hope to achieve faster or stronger response, is destructive to the natural immune system. Furthermore, cortisone may be used in children and an adult dose in this case may be excessive. Consequently, it is important not only to administer verifiable amounts of these hormones, but also make abuse by repeated dosing or administration of adult dose to a child as inconvenient as possible. Administration of steroid hormones in suggested manner on discs of filtration paper can achieve that objective. Paper discs are easily made in different sizes, just fitting the mouth of a child or an adult. Full elution of a paper disc takes up to five minutes, and if only one such disc fits into a mouth, to administer high doses used by athletes abusing testosterone may require up to an hour time. Thus incorrect use or abuse of steroid hormones may be made preventable by an appropriate size of the paper disc.

According to one embodiment of the invention, the pharmaceutical agent is cortisone and the amount of cortisone is an amount that is clinically effective. For example, the amount of cortisone or of its synthetic potent analogue may be from 2 to 100 mg. The amount of agent used depends greatly on the potency of the corticoide compound used.

According to one embodiment of the invention the pharmaceutical agent is testosterone and the amount of testosterone in the pharmaceutical product is 0.1 - 5 mg, preferably 0.3 - 3 mg, more preferably 0.4 - 2 mg. These low amounts are sufficient to produce a hormone pulse that enhances the overall well-being of the male subjects.

According to another embodiment of the invention the pharmaceutical agent is estradiol and the amount of estradiol is 0.01 - 0.1 mg, preferably 0.015 - 0.05 mg, more preferably 0.02 - 0.04 mg. This embodiment is especially suitable for hormone supplementation to female subjects.

According to one embodiment of the present invention the pharmaceutical product is in form of a sublingual administration formula. Especially preferred are sublingual administration forms, where the hormone is applied or absorbed on an absorbing matrix, such as absorbing paper i.e. filtration paper. The matrix may or may not disintegrate in water. As the sex hormone is complexed with cyclodextrin derivative, the hormone is highly water soluble and will easily be absorbed through oral mucosa.

Preferably the period during which the sex hormone is supplied through oral mucosa into the circulation is as short as possible. By using sublingual administration, a well-defined and controlled entry can be achieved. The area from which absorption of the hormone occurs is highly vascular and does not contain tissues, which would slow down transfer of the hormone into circulation. Any solubilised hormone that may be accidentally swallowed is not transferred into general circulation, but is metabolized by liver. That, in conjunction with the constant formation of saliva, results in ending of the absorption process in a well-defined manner.

According to one embodiment of the invention the pharmaceutical product is especially suitable for counteracting adverse effects caused by decreasing sex hormone levels at ageing. Low levels of sex hormone, such as testosterone, may result in reduced muscular strength and may cause depression or apathy.

Muscular weakness, together with diminished sense of balance may lead to accidental falls, which can thus be prevented by the use of the pharmaceutical composition according to the invention. The present invention is especially suitable for treating signs of ageing in male subjects.

The pharmaceutical product according to the present invention can also be used to enhance the female sex hormone levels after childbirth. Often a decline in sex hormone level is caused by natural postnatal changes. These low hormone levels may result in postnatal depression or in general indisposition. The present invention can be used to counteract these adverse effects. As the hormone levels employed in the present invention are low, they are safe for the nursing female and the child. The present invention is also suitable for treating the symptoms of female menopause.

According to an embodiment of the present invention the pharmaceutical product can be used for the normalisation of skin in aging men. Low testosterone levels may cause flabbiness and increased drying of the skin. The composition according to the present invention can be used to prevent or treat this condition.

Sex hormone level in a human is subject to roughly quotidian cycles. Therefore it is natural if supplement comprising the sex hormone in levels according to the invention is administered once or twice a day. Administration can be also three, four or five times a day, if the single dose is kept is at low level. One of the advantages of the present invention is that the administration can be done in a manner that imitates the body's natural hormone level fluctuation and avoids the overdoses.

The invention also relates to the use of a water-soluble complex of cortisone and a cyclodextrin derivative for manufacture of an oral transmucosal medicament for treatment of an allergic reaction and/or anaphylactic shock, said medicament comprising cortisone or of its synthetic potent analogue in an amount that is clinically effective. This use is especially beneficial for persons having severe allergic reactions and being far from hospitals.

The present invention also relates to a pharmaceutical product for the treatment of hormone depletion, wherein said product is adapted for oral transmucosal administration comprising a water-soluble complex of a sex hormone, selected from the group consisting of estradiol and testosterone, and a cyclodextrin derivative, wherein the amount of the sex hormone in the composition is 0.01 - 7 mg, and wherein said product is in the form of filtration paper for sublingual use into which filtration paper said complex has been absorbed.

The present invention further relates to a pharmaceutical product for the treatment of allergic reactions and/or anaphylactic shock, wherein said product is adapted for oral transmucosal administration comprising a water-soluble complex of cortisone or of its synthetic potent analogue and a cyclodextrin derivative, wherein the amount of cortisone or of its synthetic potent analogue in the composition is an amount that is clinically effective, and wherein said product is in the form of filtration paper for sublingual use into which filtration paper said complex has been absorbed.

The present invention is illustrated by the following examples that should not be considered limiting.

### EXPERIMENTAL PART

### EXAMPLE 1

The purpose of experiment in Example 1 is to ascertain if the above administration of testosterone to man can be performed safely. Adult male produces roughly 25 mg of testosterone during the whole day, and to enter that amount of testosterone into the circulation in a few minutes may have undesired consequences.

For preparation of sublingual pharmaceutical form of testosterone the process delineated in U.S. patent 6,884,790 B2 is used. Hydroxypropyl beta-cyclodextrin, pharmaceutical grade, 12 parts, is dissolved in water to form 50 % w/w solution, then 1 part of testosterone is added and the suspension stirred for two days. Nearly all testosterone is dissolved at this time and solution is fully clarified by filtration. Testosterone concentration in the solution is determined by spectrophotometry. To prepare the required pharmaceutical form a volume of the solution containing the desired dose of testosterone is deposited on disk of filtration paper about 2 cm in diameter and left to dry fully at room temperature. Small droplet of diluted solution of food dye is spotted on the rim of the disk and left also to dry fully.

Subjects are instructed to insert the disc under tongue and keep it there for three to five minutes, then check whether dye spot disappeared. If that is so, the water-soluble content of the disc is eluted fully, and the disc is discarded.

Few healthy elderly men undergo blood testing. Subjects are administered sublingually 5 mg testosterone using the above specified paper disks. This dose represents more than double of the expected recommended daily dose of testosterone supplementation. The dose is administered in mornings of two subsequent days. Two days after the administration subjects again undergo blood screening testing.

Subjects do not observe any ill effects. Blood tests results do not indicate any untoward effects. Following parameters are evaluated: lipoprotein cholesterol fractions, follicle stimulating hormone (FSH) and luteinizing hormone (LH) concentrations, liver function indicators and sex-hormone binding globulin (SHBG) concentration changes. Conclusion is that to human amount of testosterone envisioned to satisfy the need of one day can be safely administered as a single dose.

### EXAMPLE 2

The purpose of Example 2 is to ascertain bioefficacy of once a day of sublingual dose of testosterone. Of interest are scores of physical stamina, scores of sexual motivation and scores of mood elevation. Since all these parameters are evaluated subjectively, subjects do not know whether disks are placebo or contain hormone. To eliminate individual variation cross over design is used. Each subject self-administers each morning a disc. These discs are of the same kind, i.e. placebo or testosterone containing, for a week. The following week subject receives discs of the other kind.

Subjects score their impressions on scale one to three - one being no change, two weakly felt improvement, and three noticeably felt improvement. Mood elevation score is recorded for period within two hours of the administration, score for physical stamina is recorded after standard exercise performed in afternoon of the administration and scores for sexual motivation apply to 24-hour period after administration.

Subjects, even when given medication in blind manner, can distinguish placebo discs and discs with medication just by their bioeffects. Overall positive feeling of the subject treated with discs containing testosterone tend to increase with the length of the treatment.

## Claims

1. A pharmaceutical product adapted for oral transmucosal administration in the form of filtration paper for sublingual use comprising a water-soluble complex of a pharmaceutical agent and a cyclodextrin derivative adsorbed on said filtration paper, wherein the pharmaceutical agent is
- estradiol or testosterone in an amount of 0.01 - 7 mg, or
- cortisone or a synthetic potent analogues of cortisone in an amount that is clinically effective.

2. Pharmaceutical product according to claim 1, **characterised in that** the pharmaceutical agent is cortisone.

3. Pharmaceutical product according to claim 1, **characterised in that** the pharmaceutical agent is testosterone and the amount of testosterone is 0.1 - 5 mg, preferably 0.3 - 3 mg.

4. Pharmaceutical product according to claim 1, **characterised in that** the s pharmaceutical agent is estradiol and the amount of estradiol is 0.01 - 0.1 mg, preferably 0.015 - 0.05 mg.

5. Use of a water-soluble complex of a sex hormone and a cyclodextrin derivative for manufacture of an oral sublingual supplement in the form of a filtration paper into which filtration paper said complex has been absorbed, for treatment of hormone depletion, said medicament comprising 0.01 - 7 mg of hormone and said sex hormone being selected from the group consisting of estradiol and testosterone.

6. Use according to claim 5, **characterised in that** the hormone depletion is associated with ageing.

7. Use according to claim 5, **characterised in that** the hormone depletion is caused by natural postnatal changes or menopause.

8. Use according to claim 5 or 6, **characterised in that** the sex hormone is testosterone and the amount of the hormone is 0.1 - 5 mg, preferably 0.3 - 3 mg.

9. Use according to claim 5 or 7, **characterised in that** the sex hormone is estradiol and the amount of the hormone is 0.01 - 0.1 mg, preferably 0.015 - 0.05 mg.

10. Use according to any of the preceding claims 5-9, **characterised in that** the supplement is administered once or twice a day.

11. Use of a water-soluble complex of cortisone or of its synthetic potent analogue and a cyclodextrin derivative for manufacture of an oral transmucosal medicament for treatment of an allergic reaction and/or anaphylactic shock, said medicament comprising cortisone or its synthetic potent analogue in a a clinically effective dose.
